(19) Europäisches Patentamt / European Patent Office / Office européen des brevets

(11) EP 4 197 994 B1

(12) EUROPÄISCHE PATENTSCHRIFT

(45) Veröffentlichungstag und Bekanntmachung des Hinweises auf die Patenterteilung: **11.09.2024 Patentblatt 2024/37**

(21) Anmeldenummer: **21216065.9**

(22) Anmeldetag: **20.12.2021**

(51) Internationale Patentklassifikation (IPC):
***C07C 29/151*** $^{(2006.01)}$ ***C07C 31/04*** $^{(2006.01)}$
***B01J 8/00*** $^{(2006.01)}$ ***C07C 29/152*** $^{(2006.01)}$

(52) Gemeinsame Patentklassifikation (CPC):
(C-Sets verfügbar)
**C07C 29/152; C07C 29/151** (Forts.)

# (54) VERFAHREN UND ANLAGE ZUR HERSTELLUNG VON METHANOL

METHOD AND SYSTEM FOR MANUFACTURED METHANOL

PROCÉDÉ ET INSTALLATION DE PRODUCTION DE MÉTHANOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung: **21.06.2023 Patentblatt 2023/25**

(73) Patentinhaber: **L'Air Liquide, Société Anonyme pour l'Etude et l'Exploitation des Procédés Georges Claude**
**75007 Paris (FR)**

(72) Erfinder:
- **Schuhmann, Timm**
  **64625 Bensheim (DE)**
- **Oelmann, Tobias**
  **60439 Frankfurt am Main (DE)**
- **Castillo-Welter, Frank**
  **60388 Frankfurt (DE)**
- **Wagner, Marc**
  **78350 Jouy-En-Josas (FR)**

(74) Vertreter: **Air Liquide**
**L'Air Liquide S.A.**
**Direction de la Propriété Intellectuelle**
**75, Quai d'Orsay**
**75321 Paris Cedex 07 (FR)**

(56) Entgegenhaltungen:
**EP-A1- 3 401 300**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

(52) Gemeinsame Patentklassifikation (CPC): (Forts.)

C-Sets
**C07C 29/151, C07C 31/04;**
**C07C 29/152, C07C 31/04**

## Beschreibung

### Gebiet der Erfindung

Die Erfindung betrifft ein Verfahren und eine Anlage zur Herstellung von Methanol im Industriemaßstab unter Verwendung einer Strahlpumpe als Mittel zum Rückführen des bei der Methanolsynthese nicht umgesetzten Synthesegases.

### Stand der Technik

Methanol wird im großtechnischen Maßstab aus Synthesegas hergestellt. Synthesegas ist ein Gemisch aus vorwiegend Wasserstoff ($H_2$) und Kohlenmonoxid (CO) sowie Kohlendioxid ($CO_2$). Darüber hinaus weist es kleinere Mengen an unter den Bedingungen der Methanolsynthese inerten Gasbestandteilen auf. Kohlenmonoxid sowie Kohlendioxid werden dabei häufig unter dem Begriff "Kohlenstoffoxide" zusammengefasst. Im heute als Niederdruck-Methanolsynthese bezeichneten Prozess wird das Synthesegas bei einem Synthesedruck von 60 bis 120 bar zu Methanol und Wasser (als zwangsläufig anfallendes Nebenprodukt) umgesetzt. Das eingesetzte Synthesegas, häufig als Frischgas (engl. *make-up gas*) bezeichnet, wird dabei nach Verdichtung auf den jeweiligen Synthesedruck bei Katalysator-Temperaturen von üblicherweise größer als 200 °C durch ein Katalysatorbett eines Methanolsynthese-Katalysators geleitet. Bei dem Methanolsynthese-Katalysator handelt es sich üblicherweise um eine als katalytisch aktive Spezies Kupfer aufweisende Zusammensetzung. Je nach Verfahrensführung kommen eine oder mehrere hintereinander oder parallel geschaltete Synthesestufen (Reaktorstufen) zum Einsatz, welche jeweils über ein entsprechendes Katalysatorbett verfügen. Dabei ist die Umsetzung der Kohlenstoffoxide zu Methanol und Wasser am Katalysator aufgrund der Einstellung eines thermodynamischen Gleichgewichts gemäß der Reaktionen

$$CO_2 + 3\ H_2 \rightleftharpoons CH_3OH + H_2O,$$

$$CO + 2\ H_2 \rightleftharpoons CH_3OH$$

nicht vollständig. Aus diesem Grund wird der Herstellungsprozess üblicherweise als Kreislaufprozess in einer sogenannten Syntheseschleife (*loop*) gefahren. Dabei wird das am Ausgang der jeweiligen Synthesestufe erhaltene Reaktionsgemisch unter den Siedepunkt von Methanol abgekühlt, um ein als Rohmethanol bezeichnetes Gemisch aus Methanol und Wasser (sowie unerwünschten kondensierbaren Nebenprodukten) aus dem Kreislauf auszuleiten. Gleichzeitig wird nicht umgesetztes Synthesegas nach Zusammenführen mit dem Frischgas zur erneuten Reaktion zum Methanolsynthese-Katalysator zurückgeführt. Von dem nicht umgesetzten Synthesegas, auch als Restgas oder Recycle-Gas bezeichnet, wird kontinuierlich ein Teilstrom als Spülgasstrom (*purge*) entnommen, um die Erhöhung der Konzentration von inerten Bestandteilen in der Syntheseschleife mit fortschreitender Zeit zu vermeiden.

Das Frischgas wird üblicherweise bei Drücken zwischen 20 und 40 bar erzeugt und für die Methanolsynthese über einen sogenannten Synthesegas- oder FrischgasKompressor auf ein höheres Druckniveau von üblicherweise deutlich mehr als 60 bar verdichtet. Das Verfahren arbeitet mit relativ hohen Gaskreisläufen über den Reaktorteil, um eine ausreichende Umsetzung nach Maßgabe der Ausbeute an zu Methanol umgesetztem Kohlenstoff und Wasserstoff zu erreichen. Die Verdichtungsarbeit für das Restgas wird dabei in der Regel mit einer zusätzlichen Verdichtungsstufe des Frischgas-Kompressors (insbesondere bei hohen Kapazitäten) oder mit einer zusätzlichen Maschine, also einem dedizierten Restgaskompressor (insbesondere bei kleinen Kapazitäten), durchgeführt. Je nach Reaktorsystem und Zusammensetzung des Frischgases ist der im Kreislauf geführte Volumenstrom des Restgases bis zu 5 mal höher als der Volumenstrom des Frischgases aus der Synthesegasproduktion. Dies ist insbesondere bei Synthesegas mit einem hohen Kohlendioxid-Gehalt der Fall, bei dem der thermodynamische Gleichgewichtsumsatz gering ist. Der Quotient aus Restgas-Volumenstrom und Frischgas-Volumenstrom wir auch als Rezirkulationsrate bezeichnet.

Durch Hinzufügen weiterer Synthesestufen einschließlich Zwischenkondensation des erhaltenen Rohmethanols kann der Umsatz "pro Durchgang" erhöht werden. Die Rezirkulationsrate kann dadurch reduziert werden. Dieses Prinzip ist beispielsweise in der EP 3 401 299 A1 und der EP 3 401 300 A1 beschrieben.

### Beschreibung der Erfindung

Der zusätzliche Kompressor oder die zusätzliche Kompressorstufe für die Verdichtung und Rückführung des Restgases zum Reaktoreinlass ist eine kostenintensive Vorrichtung, welche einen hohen Wartungsaufwand erfordert und anfällig für Störungen ist. Die in der Nähe des Reaktors angeordnete Vorrichtung benötigt ferner zusätzliche Fläche

und Baustahl für Installation und Betrieb. Weiterhin erfordert eine solche Vorrichtung eine entsprechende Instrumentierung für den Betrieb und die Kontrolle. Auch die Schmierung und Abdichtung der Vorrichtung im Sinne einer regelmäßigen Wartung ist zeit- und arbeitsaufwändig.

Eine Aufgabe der vorliegenden Erfindung besteht darin ein Verfahren und eine Anlage zur Herstellung von Methanol bereitzustellen, welche(s) die Nachteile des Standes der Technik zumindest teilweise überwindet.

Insbesondere besteht eine Aufgabe der vorliegenden Erfindung darin ein Verfahren bereitzustellen, welches das Ersetzen des Restgaskompressors oder der zusätzlichen Kompressorstufe durch ein wartungsfreundlicheres und weniger kostenintensives Bauteil ermöglicht.

Eine weitere Aufgabe der vorliegenden Erfindung besteht darin ein Verfahren bereitzustellen, welches das Ersetzen des Restgaskompressors oder der zusätzlichen Kompressorstufe durch ein wartungsfreundlicheres und weniger kostenintensives Bauteil für Anlagen im kleinen und mittleren Maßstab ermöglicht.

Eine weitere Aufgabe der vorliegenden Erfindung besteht darin ein Verfahren bereitzustellen, welches das Ersetzen des Restgaskompressors oder der zusätzlichen Kompressorstufe durch ein wartungsfreundlicheres und weniger kostenintensives Bauteil für Anlagen ermöglicht, welche das Prinzip der Multistufensynthese von Methanol mit Zwischenkondensation nutzen.

Eine weitere Aufgabe der vorliegenden Erfindung besteht darin ein Verfahren bereitzustellen, welches das Ersetzen des Restgaskompressors oder der zusätzlichen Kompressorstufe durch ein wartungsfreundlicheres und weniger kostenintensives Bauteil für Verfahren ermöglicht, welche ein Synthesegas mit hohem Kohlenmonoxidanteil nutzen.

Eine weitere Aufgabe der vorliegenden Erfindung besteht darin ein Verfahren bereitzustellen, welches das Ersetzen des Restgaskompressors oder der zusätzlichen Kompressorstufe durch ein wartungsfreundlicheres und weniger kostenintensives Bauteil für Verfahren ermöglicht, welche ein Synthesegas mit hohem Kohlendioxidanteil und das Prinzip der Mehrstufensynthese von Methanol mit Zwischenkondensation nutzen.

Eine weitere Aufgabe der vorliegenden Erfindung besteht darin eine Anlage bereitzustellen, welche zumindest eine der vorgenannten Aufgaben zumindest teilweise löst.

Ein Beitrag zur mindestens teilweisen Erfüllung mindestens einer der obigen Aufgaben wird durch die unabhängigen Ansprüche geleistet. Die abhängigen Ansprüche stellen bevorzugte Ausführungsformen bereit, die zur mindestens teilweisen Erfüllung mindestens einer der Aufgaben beitragen. Bevorzugte Ausgestaltungen von Bestandteilen einer erfindungsgemäßen Kategorie sind, soweit zutreffend, ebenso bevorzugt für gleichnamige oder entsprechende Bestandteile einer jeweils anderen erfindungsgemäßen Kategorie.

Die Ausdrücke "aufweisend", "umfassend" oder "beinhaltend" etc. schließen nicht aus, dass weitere Elemente, Inhaltsstoffe etc. enthalten sein können. Der unbestimmte Artikel "ein" schließt nicht aus, dass eine Mehrzahl vorhanden sein kann.

Die vorgenannten Aufgaben werden zumindest teilweise gelöst durch ein Verfahren zur Herstellung von Methanol, wobei das Verfahren die folgenden Schritte umfasst:

a) Bereitstellen und Verdichten eines Frischgasstroms MG, wobei der Frischgasstrom MG zumindest ein Kohlenstoffoxid und Wasserstoff aufweist;
b) Umsetzen eines Synthesegasstroms SG in einer Synthesestufe an einem festen Methanolsynthese-Katalysator, wobei Rohmethanol und ein Restgasstrom RG erhalten werden, und wobei das Rohmethanol nach Kühlung und Kondensation als flüssiges Reaktionsprodukt und der Restgasstrom RG aus der Synthesestufe ausgeleitet werden;
c) Zusammenführen des Restgasstroms RG mit dem Frischgasstrom MG, wobei der Synthesegasstrom SG erhalten wird,

dadurch gekennzeichnet, dass
der Restgasstrom RG und der Frischgasstrom MG mittels einer Strahlpumpe zusammengeführt werden, wobei der verdichtete Frischgasstrom MG mit einem Druck $p_1$ der Strahlpumpe über deren Treibmedienanschluss als Treibmedium zugeführt wird, und der Restgasstrom RG mit einem Druck $p_3$ der Strahlpumpe über deren Saugstutzen als Saugmedium zugeführt wird, und wobei der Synthesegasstrom SG mit einem Druck $p_2$ aus der Strahlpumpe über deren Druckstutzen ausgeleitet und anschließend der Synthesestufe zugeführt wird, und wobei $p_1 > p_2 > p_3$ gilt.

Erfindungsgemäß wird der Restgaskompressor oder die zusätzliche Kompressorstufe durch eine Strahlpumpe ersetzt. Strahlpumpen sind dem Fachmann bekannte Apparate zum Fördern, Verdichten oder Mischen von Gasen, Flüssigkeiten oder Feststoffen. Es handelt sich dabei um Pumpen ohne bewegliche Teile, welche ihre Arbeit dadurch leisten, dass Druckenergie in Geschwindigkeit umgesetzt wird. Das Grundprinzip besteht darin, dass aus einer Düse ein flüssiger oder gasförmiger Strahl mit hoher Geschwindigkeit austritt, welcher eine Flüssigkeit, ein Gas oder einen Feststoff aus seiner Umgebung mitreißt und beschleunigt. Daraus resultiert ein Gemisch aus treibendem und mitgerissenem (angesaugtem) Stoff, dessen Geschwindigkeit in einer zweiten Düse unter Druckerhöhung wieder herabgesetzt wird.

Eine Strahlpumpe, insbesondere die erfindungsgemäße Stahlpumpe, kann auch als Ejektor bezeichnet werden.

Insbesondere handelt es sich bei der Strahlpumpe oder dem Ejektor gemäß der Erfindung um eine sogenannte Gasstrahlpumpe, da ausschließlich gasförmige Stoffe gefördert werden. Eine solche Gasstrahlpumpe kann auch als Gasstrahl-Verdichter bezeichnet werden.

Die Strahlpumpe gemäß der vorliegenden Erfindung umfasst einen Treibmedienanschluss, einen Saugstutzen und einen Druckstutzen. Der verdichtete Frischgasstrom MG dient als Treibmedium oder Treibmittel, wird daher der Strahlpumpe über deren Treibmedienanschluss zugeführt. Hinter dem Treibmedienanschluss der Strahlpumpe ist üblicherweise eine Treibdüse angeordnet. Die Treibdüse beschleunigt das Treibmedium, hier den verdichteten Frischgasstrom MG, und erzeugt einen Niederdruckbereich. Dadurch wird das über den Saugstutzen angesaugte Saugmedium, hier der Restgasstrom RG, mitgerissen. Das mitgerissene Saugmedium wird dabei durch das Treibmedium verdichtet. Treibmedium und Saugmedium werden üblicherweise innerhalb der im Kopfbereich der Strahlpumpe angeordnete Mischkammer der Strahlpumpe gemischt. Das resultierende Gemisch aus treibendem und mitgerissenem Gas, hier das Synthesegas SG, tritt anschließend über den Druckstutzen der Stahlpumpe aus der Strahlpumpe aus. Zwischen Mischkammer und Druckstutzen der Strahlpumpe ist insbesondere ein Diffusor angeordnet. Innerhalb des Diffusors nimmt die Geschwindigkeit ab und der Druck wird wieder hergestellt. Der Strömungskanal des Diffusors besteht dabei insbesondere aus einem sich in Strömungsrichtung verengenden Teil, dem Einlaufkonus, einem zylindrischen Stück, der Mischdüse, und einem sich erweiternden Teil, dem Auslaufkonus. Maßgebend für die Funktion der Strahlpumpe sind die an ihren Anschlüssen herrschenden Drücke und zugehörige Massenströme.

Dabei wird der verdichtete Frischgasstrom MG der Strahlpumpe mit einem Druck $p_1$ über deren Treibmedienanschluss zugeführt. Bei dem Druck $p_1$ handelt es sich somit um den sogenannten Treibmediendruck vor dem oder am Treibmedienanschluss. Der Restgasstrom RG wird der Strahlpumpe mit einem Druck $p_3$ über deren Saugstutzen zugeführt. Bei dem Druck $p_3$ handelt es sich somit um den sogenannten Saugdruck vor dem oder am Saugstutzen. Der Synthesegasstrom SG wird aus der Strahlpumpe mit einem Druck $p_2$ über deren Druckstutzen ausgeleitet. Bei dem Druck $p_2$ handelt es sich somit um den sogenannten Gegendruck hinter dem oder am Druckstutzen. Dabei gilt $p_1 > p_2 > p_3$, das heißt der Treibmediendruck ist stets am höchsten, der Saugdruck ist stets am niedrigsten und der Gegendruck liegt stets zwischen dem Treibmediendruck und dem Saugdruck.

Da der Gegendruck $p_2$ am Druckstutzen der Strahlpumpe höher ist als der Saugdruck $p_3$ wird der Restgasstrom RG quasi mit Hilfe des verdichteten Frischgasstroms MG verdichtet.

Im Sinne des erfindungsgemäßen Verfahrens ist somit lediglich ein Frischgaskompressor zum Verdichten des bereitgestellten Frischgasstroms MG erforderlich. Der verdichtete Frischgasstrom MG weist nach dem Verdichten einen Druck von zumindest $p_1$ auf. Der Druck $p_3$ entspricht mindestens dem Synthesedruck, mit dem der Synthesegasstrom SG der Synthesestufe zugeführt wird. Ein dedizierter Restgaskompressor (Recyclegaskompressor) oder eine zusätzliche Kompressorstufe am Frischgaskompressor ist gemäß dem erfindungsgemäßen Verfahren somit nicht erforderlich. Gemäß einer Ausführungsform beinhaltet das erfindungsgemäße Verfahren daher keinen Schritt zum Verdichten des Restgasstroms RG in einer (dedizierten) Restgaskompressorstufe. Gemäß einer weiteren Ausführungsform beinhaltet das erfindungsgemäße Verfahren nur eine einzige Verdichtungsstufe eines Frischgaskompressors zum Verdichten des Frischgasstroms MG.

Die Verwendung einer Strahlpumpe hat den Vorteil, dass Strahlpumpen keine bewegten oder beweglichen Teile aufweisen, insbesondere im Gegensatz zu den üblicherweise verwendeten Kompressoren keine rotierenden Teile aufweisen. Sie sind dadurch besonders wartungsarm. Ferner erfordern Strahlpumpen keine separate Energiequelle, wie zum Beispiel elektrische Energie oder Wärmeenergie. Vielmehr arbeiten Strahlpumpen allein auf Basis des Prinzips der Umwandlung von Druckenergie in kinetische Energie und umgekehrt.

Auch wenn der Leistungsbedarf für die Verdichtung des Frischgasstroms MG durch den Frischgaskompressor bei Verwendung einer Strahlpumpe zum Verdichten des Restgasstroms MG steigt (höhere Betriebskosten), so wird dieser Nachteil durch die Verwendung einer Strahlpumpe zum Verdichten des Restgasstroms RG in dem Sinne überkompensiert, dass auf einen zusätzlichen Restgaskompressor oder eine zusätzliche Kompressorstufe für den Frischgaskompressor verzichtet werden kann (niedrigere Investitions- und Wartungskosten). Dies trifft insbesondere für Verfahren und Anlagen zu, welche kleinere und mittlere Kapazitäten aufweisen und dadurch eine niedrigere Methanolproduktionsmenge pro Zeiteinheit aufweisen als Großanlagen. Bei kleineren und mittleren Anlagen ist es insbesondere von Vorteil, wenn aufgrund eines im Verhältnis zu Großanlagen niedrigeren zu erwartenden Umsatzes die Investitions- und Wartungskosten gesenkt werden können.

Der Frischgasstrom MG weist zumindest ein Kohlenstoffoxid und Wasserstoff auf. Unter einem "Kohlenstoffoxid" ist dabei entweder Kohlenmonoxid oder Kohlendioxid zu verstehen. Der Frischgasstrom MG weist somit zumindest

- Kohlenmonoxid und Wasserstoff, oder
- Kohlendioxid und Wasserstoff, oder
- Kohlenmonoxid, Kohlendioxid und Wasserstoff

auf. Ferner weist der Frischgasstrom gegebenenfalls unerwünschte Bestandteile, insbesondere unter den Bedingungen

der Methanolsynthese inerte Bestandteile wie Methan und/oder Stickstoff auf. Der Frischgasstrom MG wird insbesondere durch ein dem Fachmann bekanntes Verfahren zur Erzeugung von Synthesegas aus einerfossilen Kohlenstoffquelle bereitgestellt, wie zum Beispiel Dampfreformierung (SMR), autotherme Reformierung (ATR), partielle Oxidation (POx), Kohlevergasung oder Kombinationen damit oder daraus. Der Synthesegasstrom kann ferner zumindest teilweise aus einer nicht fossilen Kohlenstoffquelle stammen. So kann Synthesegas beispielsweise durch Vergasung von Biomasse oder kommunalen Abfällen erzeugt werden. Auch die "Herstellung" von Synthesegas durch Zusammenführen von "grünem" Wasserstoff, beispielsweise aus der Elektrolyse von Wasser, und Kohlendioxid aus einer Verbrennungsanlage, beispielsweise einer Müllverbrennungsanlage, ist denkbar.

Am Methanolsynthese-Katalysator erfolgt die Umsetzung des Synthesegasstroms SG zu Rohmethanol. Unter "Rohmethanol" wird dabei ein Gemisch aus Methanol und Wasser verstanden, welches ferner nicht kondensierbare und kondensierbare unerwünschte Nebenprodukte enthalten kann. Die Umsetzung erfolgt in einer Syntheseschleife, das heißt am Katalysator nicht umgesetztes Synthesegas aus dem Synthesegasstrom SG wird als Restgasstrom RG zurückgeführt und mittels der Strahlpumpe mit frischem Synthesegas (Frischgas) des Frischgasstroms MG gemischt. Die Umsetzung des Synthesegasstroms SG am Methanolsynthese-Katalysator erfolgt vorzugsweise bei einer Katalysatortemperatur von 220 °C bis 270 °C und vorzugsweise einem Druck von 55 bar bis 80 bar. Das Frischgas weist vor der Verdichtung durch einen Frischgaskompressor beispielsweise einen Druck von 20 bar bis 40 bar auf.

Zur Abtrennung des Rohmethanols vom Restgasstrom RG erfolgt eine Kühlung des Rohmethanol und Restgas aufweisenden Produktgemischs vorzugsweise unter den Taupunkt von Methanol. Dadurch kondensiert das Rohmethanol und kann dadurch vom weiterhin gasförmigen Restgasstrom RG abgetrennt werden. Rohmethanol wird somit nach Kühlung und Kondensation (und Abtrennung vom gasförmigen Restgasstrom RG) als flüssiges Reaktionsprodukt aus der Synthesestufe ausgeleitet. Ebenso wird der Restgasstrom RG als vom flüssigen Reaktionsprodukt getrennter gasförmiger Strom aus der Synthesestufe ausgeleitet.

Eine bevorzugte Ausführungsform des erfindungsgemäßen Verfahrens ist dadurch gekennzeichnet, dass für eine Rezirkulationsrate R, definiert als

$$R = \frac{Volumenstrom\ (RG)}{Volumenstrom\ (MG)},$$

$0{,}3 \leq R \leq 2{,}0$ gilt, vorzugsweise $0{,}5 \leq R \leq 1{,}5$ gilt, weiter bevorzugt $1{,}0 \leq R \leq 1{,}5$ gilt.

Die Rezirkulationsrate R ist definiert als der Quotient aus dem Volumenstroms des Restgasstroms RG und dem Volumenstrom des Frischgasstroms MG. Über die Rezirkulationsrate R bestimmt sich somit, wie viel in der Synthesestufe nicht umgesetztes Restgas und wie viel verdichtetes Frischgas im Synthesegasstrom SG vorhanden ist.

Eine Rezirkulationsrate R von 0,3 bis 2,0, vorzugsweise 0,5 bis 1,5, weiter bevorzugt 1,0 bis 1,5, ist verhältnismäßig niedrig und lässt sich insbesondere dann realisieren, wenn das thermodynamische Gleichgewicht der Reaktion des Synthesegasgemischs zu Rohmethanol stärker auf der Produktseite liegt. In diesem Fall muss nur ein verhältnismäßig kleiner Teil des Restgasstroms RG zur Synthesestufe zurückgeführt werden. Der Leistungsbedarf der Verdichtungsarbeit bestimmt sich in diesem Fall hauptsächlich über die Verdichtung des Frischgasstroms zum Frischgasstrom MG und weniger über die Verdichtung des Restgasstroms RG. Daher kann insbesondere bei niedrigen Rezirkulationsraten R wie den vorgenannten auf einen dedizierten Restgaskompressor verzichtet werden, und die Verdichtungsarbeit bezüglich des Restgasstroms RG stattdessen wie oben beschrieben mittels einer Strahlpumpe realisiert werden.

Niedrige Rezirkulationsraten wie die vorgenannten lassen sich insbesondere auch bei einstufigen Methanolsynthesen realisieren für den Fall, dass der Anteil an Kohlenmonoxid im Kohlenoxid-Gemisch besonders hoch ist, so dass das Reaktionsgleichgewicht stark auf der Produktseite liegt. Dies ist beispielsweise bei Synthesegas aus Kohlevergasung der Fall. Gemäß einer entsprechenden Ausführungsform liegt der Anteil an Kohlenmonoxid an den Kohlenoxiden im Frischgasstrom MG bei mindestens 50 Vol.-%, oder bei mindestens 75 Vol.-%, oder bei mindestens 90 Vol.-%.

Eine bevorzugte Ausführungsform des erfindungsgemäßen Verfahrens ist dadurch gekennzeichnet, dass das Verfahren eine Vielzahl N in Serie geschalteter Synthesestufen beinhaltet, wobei das Rohmethanol nach Kühlung und Kondensation als flüssiges Reaktionsprodukt aus jeder der Synthesestufen ausgeleitet wird, und der Restgasstrom RG aus der letzten der N Synthesestufen ausgeleitet wird.

Gemäß dieser bevorzugten Ausführungsform beinhaltet das Verfahren eine Vielzahl Synthesestufen, also mindestens zwei Synthesestufen. Dabei sind diese Synthesestufen in Serie geschaltet, und jede der Synthesestufen beinhaltet eine Zwischenkondensation zum Ausleiten von Rohmethanol aus der jeweiligen Synthesestufe. Mit anderen Worten, nach jeder der N Synthesestufen erfolgt eine Kühlung und Kondensation zum Ausleiten des Rohmethanols aus der jeweiligen Synthesestufe. Der gleichzeitig anfallende Strom an nicht umgesetztem Synthesegas je Synthesestufe wird dabei in die jeweils stromabwärts angeordnete Synthesestufe überführt, sofern es sich nicht um die letzte der N Synthesestufen handelt. Der in die jeweils stromabwärts angeordnete Synthesestufe überführte Gasstrom wird im Sinne der vorliegenden Erfindung als Zwischengasstrom IG bezeichnet. So wird beispielsweise das nicht umgesetzte Synthesegas der ersten Synthesestufe eines Verfahrens mit drei Synthesestufen als erster Zwischengasstrom IG 1 in die zweite Synthesestufe überführt und dort weiter umgesetzt. Das nicht umgesetzte Synthesegas der zweiten Synthesestufe des Verfahrens wird als zweiter Zwischengasstrom IG 2 in die dritte Synthesestufe überführt und dort weiter umgesetzt.

Weist das Verfahren also allgemein gesprochen eine Zahl N Synthesestufen auf, so weist das Verfahren entsprechend eine Zahl von N-1 Zwischengasströmen IG auf. Dabei wird der Zwischengasstrom einer n-ten Synthesestufe der N Synthesestufen in die jeweils (n+1)te Synthesestufe überführt und dort weiter umgesetzt, außer es handelt sich bei der n-ten Synthesestufe um die letzte der in Serie angeordneten N Synthesestufen.

Nicht umgesetztes Synthesegas der dritten oder allgemein gesprochen letzten der N Synthesestufen entspricht dem Restgasstrom RG, welcher mit dem Frischgasstrom MG mittels der Strahlpumpe zusammengeführt wird. Ferner ist es auch möglich, dass ein Teil eines Zwischengasstroms IG oder ein Teil mehrerer der Zwischengasströme IG mit dem Frischgasstrom MG zusammengeführt wird/werden.

Der Kohlenstoffumsatz "pro Durchgang" ist bei einer gemäß dieser Ausführungsform definierten Mehrstufensynthese stets höher als bei einer einstufigen Synthese. Entsprechend ist der Volumenstrom des Restgasstroms RG niedriger. Dies gilt entsprechend für die Rezirkulationrate R, welche für gleiches Ausgangsmaterial (Zusammensetzung des Frischgasstroms MG) stets niedriger ist als bei einer einstufigen Synthese. Entsprechend kommen die vorgenannten Vorteile der Verwendung einer Strahlpumpe zum Verdichten des Restgasstroms RG bei niedrigen Rezirkulationsraten insbesondere bei einer Mehrstufensynthese zum Tragen, da eine niedrige Rezirkulationsrate R einer Mehrstufensynthese immanent ist.

Dabei ist eine bevorzugte Ausführungsform des erfindungsgemäßen Verfahrens dadurch gekennzeichnet, dass für die Vielzahl N in Serie geschalteter Synthesestufen $N \geq 2$ gilt, vorzugsweise $2 \leq N \leq 5$ gilt, weiter bevorzugt $3 \leq N \leq 4$ gilt.

Es ist somit bevorzugt, dass das erfindungsgemäße Verfahren 2 bis 5 in Serie geschaltete Synthesestufen umfasst, und vorzugsweise 3 oder 4 Synthesestufen umfasst. Zusätzliche Synthesestufen mit integrierter Produktabtrennung, also Zwischenkondensation des Produktgemischs und dessen Abtrennung, verringern den erforderlichen Volumenstrom an Restgas (Restgasstrom RG) bei gleichbleibender Gesamtproduktion in Bezug auf ein einstufiges Verfahren als Referenz. Dies ist bezüglich der erfindungsgemäßen Verwendung einer Strahlpumpe von Vorteil, da wie oben beschrieben die Verdichtungsarbeit für den Restgasstrom RG vornehmlich mittels der Strahlpumpe realisiert werden kann. Mit zunehmender Anzahl der Synthesestufen steigt jedoch der Gesamtdruckverlust in der Syntheseschleife, was wiederum zusätzliche Verdichtungsarbeit in Bezug auf den Restgasstrom RG bedeutet und die Verwendung einer Strahlpumpe in Abhängigkeit von weiteren Randparametern des Systems dann gegebenenfalls nicht von Vorteil. Daher ist die Verwendung einer mittleren Anzahl an Synthesestufen, insbesondere 2 bis 5 Synthesestufen und bevorzugt 3 oder 4 Synthesestufen diesbezüglich von Vorteil. Insbesondere ist dies dann vor Vorteil, wenn diese Zahl von Synthesestufen mit den vorgenannten niedrigen Rezirkulationsraten R von $0,3 \leq R \leq 2,0$, vorzugsweise $0,5 \leq R \leq 1,5$, weiter bevorzugt $1,0 \leq R \leq 1,5$ kombiniert werden.

Eine bevorzugte Ausführungsform des erfindungsgemäßen Verfahrens ist dadurch gekennzeichnet, dass der Anteil an Kohlendioxid an den Kohlenoxiden im Frischgasstrom MG mindestens 50 Vol.-% beträgt, oder mindestens 75 Vol.-% beträgt, oder mindestens 90 Vol.-% beträgt.

Kohlendioxidreiches Synthesegas wird insbesondere in mehrstufigen Methanolsynthesen verwendet, da das thermodynamische Gleichgewicht der Methanolbildungsreaktion in diesem Fall stark auf der Eduktseite liegt. Um trotzdem einen hohen Kohlenstoffumsatz zu erzeugen, wird in diesem Fall daher häufig auf eine Mehrstufensynthese mit zwei oder mehr Stufen und Zwischenkondensation je Stufe zurückgegriffen. Auch bei Verwendung von kohlendioxidreichen Synthesegasen oder gar kohlenmonoxidfreien Synthesegasen kann so eine niedrige Rezirkulationsrate R wie oben definiert realisiert werden. Die Verwendung einer Strahlpumpe gemäß Erfindung weist bei niedrigen Rezirkulationsraten R wie oben beschrieben spezifische Vorteile auf.

Die vorgenannten Aufgaben werden ferner zumindest teilweise gelöst durch eine Anlage zur Herstellung von Methanol, wobei die Anlage folgende untereinander in Wirkverbindung stehende Anlagenkomponenten aufweist:

a) Einen Kompressor zum Verdichten eines Frischgasstroms MG, wobei der Frischgasstrom MG zumindest ein Kohlenstoffoxid und Wasserstoff aufweist;
b) Eine Reaktionsvorrichtung zum Umsetzen eines Synthesegasstroms SG in einer Reaktorstufe an einem festen Methanolsynthese-Katalysator, wobei Rohmethanol und ein Restgasstrom RG durch die Umsetzung an dem Methanolsynthese-Katalysator erhältlich sind, und wobei die Reaktorstufe Mittel zum Kühlen und Kondensieren des Rohmethanols aufweist, sowie Mittel zum Ausleiten des Rohmethanols als flüssiges Reaktionsprodukt und des Restgasstroms aus der Reaktorstufe aufweist;
c) Mittel zum Zusammenführen des Restgasstroms RG und des Frischgasstroms MG, wodurch der Synthesegasstrom SG erhältlich ist,

dadurch gekennzeichnet, dass

die Anlage eine Strahlpumpe als Mittel zum Zusammenführen des Restgasstroms RG und des Frischgasstrom MG aufweist, und die Anlage Mittel zum Zuführen des verdichteten Frischgasstroms MG mit einem Druck $p_1$ zu einem Treibmedienanschluss der Strahlpumpe aufweist, wodurch der Frischgasstrom MG als Treibmedium der Strahlpumpe nutzbar ist, und die Anlage Mittel zum Zuführen des Restgasstroms RG mit einem Druck $p_3$ zu einem Saugmedienanschluss der Strahlpumpe aufweist, und der Synthesegasstrom SG mit einem Druck $p_2$ über einen Druckstutzen der Strahlpumpe aus der Strahlpumpe ausleitbar und der Reaktionsvorrichtung zuführbar ist, und wobei $p_1 > p_2 > p_3$ gilt.

Bezüglich der erfindungsgemäßen Anlage, welche auch zur Durchführung des erfindungsgemäßen Verfahrens geeignet ist, wird anstatt des für das erfindungsgemäße Verfahren benutzten Ausdrucks "Synthesestufe" der Begriff "Reaktorstufe" verwendet. Die zur Umsetzung des Synthesegasstroms SG zu Rohmethanol verwendete Reaktionsvorrichtung kann auch als Reaktor bezeichnet werden und mehrere solcher Reaktorstufen umfassen.

Eine bevorzugte Ausführungsform der erfindungsgemäßen Anlage ist dabei dadurch gekennzeichnet, dass die Reaktionsvorrichtung der Anlage eine Vielzahl P von in Serie geschalteten Reaktorstufen aufweist, wobei das Rohmethanol nach Kühlung und Kondensation als flüssiges Reaktionsprodukt aus jeder der Reaktorstufen ausleitbar ist, und der Restgasstrom RG aus der letzten der P Reaktorstufen ausleitbar ist.

Die Vielzahl P in Serie geschalteter Reaktorstufen entspricht dabei der Vielzahl N in Serie geschalteter Synthesestufen des erfindungsgemäßen Verfahrens.

Eine bevorzugte Ausführungsform der erfindungsgemäßen Anlage ist dabei dadurch gekennzeichnet, dass für die Vielzahl P Reaktorstufen $P \geq 2$ gilt, vorzugsweise $2 \leq P \leq 5$ gilt, weiter bevorzugt $3 \leq P \leq 4$ gilt.

Die erfindungsgemäße Anlage ist insbesondere so konfiguriert, dass die Anlage mit einer Rezirkulationsrate R, definiert als

$$R = \frac{Volumenstrom\ (RG)}{Volumenstrom\ (MG)},$$

betreibbar ist und $0{,}3 \leq R \leq 2{,}0$ gilt, vorzugsweise $0{,}5 \leq R \leq 1{,}5$ gilt, weiter bevorzugt $1{,}0 \leq R \leq 1{,}5$ gilt.

**Ausführungsbeispiele**

Die Erfindung wird im Folgenden durch zwei erfindungsgemäße Beispiele näher erläutert, ohne den Gegenstand der Erfindung dadurch zu beschränken. Weitere Merkmale, Vorteile und Anwendungsmöglichkeiten der Erfindung ergeben sich aus der nachfolgenden Beschreibung der Ausführungsbeispiele in Zusammenhang mit den Zeichnungen.

Es zeigt

Figur 1 ein Blockfließbild eines erfindungsgemäßen Verfahrens oder einer erfindungsgemäßen Anlage gemäß einem ersten Beispiel der Erfindung, wobei das Verfahren eine einzige Synthesestufe umfasst, und

Figur 2 ein Blockfließbild eines erfindungsgemäßen Verfahrens oder einer erfindungsgemäßen Anlage gemäß einem zweiten Beispiel der Erfindung, wobei das Verfahren drei Synthesestufen umfasst.

Figur 1 zeigt ein stark vereinfachtes Blockfließbild eines erfindungsgemäßen Verfahrens (oder einer erfindungsgemäßen Anlage) 1 gemäß einem ersten Beispiel der Erfindung. Das Verfahren gemäß Figur 1 weist nur eine einzige Synthesestufe (oder Reaktorstufe) 22 auf.

Das Verfahren umfasst eine Vorrichtung zur Synthesegaserzeugung 10, beispielsweise einen Dampfreformer. Die Vorrichtung 10 erzeugt ein Synthesegas, welches zumindest Kohlenmonoxid, Kohlendioxid, sowie Wasserstoff aufweist und als Frischgasstrom 11 bei einem Druck von 30 bar im Kompressor 12 auf 95 bar verdichtet wird. Der verdichtete Frischgasstrom MG 13 wird anschließend einer erfindungsgemäßen Strahlpumpe 14 über deren Treibmedienanschluss 15 zugeführt. Die Strahlpumpe 14 verfügt ferner über einen Saugstutzen 16 und einen Druckstutzen 17. Nach dem Eintritt des verdichteten Frischgasstroms MG 13 über den Treibmedienanschluss 15 der Strahlpumpe 14 wird dieser durch eine in der Strahlpumpe 14 vorhandene Treibdüse (nicht gezeigt) beschleunigt, wodurch im Kopfbereich der Strahlpumpe 14 ein Bereich niedrigeren Drucks erzeugt wird und dadurch ein über den Saugstutzen 16 zugeführter Restgasstrom 19 mitgerissen wird. Innerhalb der Strahlpumpe 14 wird so durch Mischung der Ströme 13 und 19 ein Synthesegasstrom 18 erzeugt, welcher am Druckstutzen 17 aus der Strahlpumpe 14 austritt. Der Synthesegasstrom 18 weist einen Druck auf, welcher niedriger ist als der Druck des verdichteten Frischgasstroms MG 13, und höher ist als der Druck des Restgasstroms RG 19. In jedem Fall weist der Synthesegasstrom SG 18 einen Druck auf, welcher mindestens so hoch ist wie der für die Methanolsynthese erforderliche Synthesedruck, welcher für die Umsetzung des Synthesegases zu Methanol in der Synthesestufe 22 erforderlich ist.

Das Verfahren umfasst ferner drei Mess- und Regelstellen zum Erfassen und Einstellen der Drücke der Ströme

13, 19 und 18, welche der Strahlpumpe 14 zugeführt oder aus dieser ausgeleitet werden. Etwaige erforderliche Regelventile oder sonstige Mess- und Regeleinrichtungen sind nicht gezeigt. Bei dem Druck $p_1$ handelt es sich um den Treibmediendruck, das heißt den Druck des verdichteten Frischgasstroms MG 13 vor dem Treibmedienanschluss 15 der Strahlpumpe 14. Bei dem Druck $p_3$ handelt es sich um den Saugdruck, das heißt den Druck des Restgasstroms RG 19 vor dem Saugstutzen 16 der Strahlpumpe 14. Bei dem Druck $p_2$ schließlich handelt es sich um den Gegendruck, das heißt den Druck des Synthesegasstroms 18 unmittelbar hinter dem Auslass, also Druckstutzen 18 der Strahlpumpe 14.

Der über den Druckstutzen 17 der Strahlpumpe 14 austretende Synthesegasstrom SG 18 wird der Synthesestufe (oder Reaktorstufe) 22 zugeführt und dort an einem festen Methanolsynthese-Katalysator zu Rohmethanol, einem Gemisch aus Methanol und Wasser sowie etwaigen unerwünschten Nebenprodukten, umgesetzt. Die Synthesestufe 22 umfasst Mittel zum Abführen der Reaktionswärme der exothermen Methanolbildungsreaktion und zum Kühlen und Kondensieren des Rohmethanols, welches anschließend durch einen Separationsschritt als Rohmethanolstrom 21 vom Restgasstrom 19 RG abgetrennt und als flüssiges Reaktionsprodukt aus der Synthesestufe 22 ausgeleitet wird. Ferner wird auch der Restgasstrom RG 19 aus der Synthesestufe 22 ausgeleitet und zum Saugstutzen der Strahlpumpe 14 zurückgeführt und so mit dem über den Treibmedienanschluss 15 zugeführten Frischgasstrom MG 13 zusammengeführt. Um die Kumulation von unter den Bedingungen der Methanolsynthese inerten Edukt- oder Produktkomponenten in der Syntheseschleife zu verhindern, wird von einem Teil des Restgasstrom RG 19 ein Strom als Purgegasstrom 20 abgeführt und einer weiteren Verwendung zugeführt.

Der Rohmethanolstrom 21 wird einer weiteren Aufarbeitung zur Gewinnung von reinem Methanol zugeführt (nicht gezeigt).

Die Rezirkulationsrate R beträgt im Falle des Beispiels gemäß Figur 1 R = 4,6.

Figur 2 zeigt ein stark vereinfachtes Blockfließbild eines erfindungsgemäßen Verfahrens (oder einer erfindungsgemäßen Anlage) 2 gemäß einem zweiten Beispiel der Erfindung. Das Verfahren gemäß Figur 2 weist drei Synthesestufen (oder Reaktorstufen) 22a, 22b und 22c auf.

Das Beispiel gemäß Figur 2 unterscheidet sich vom Beispiel gemäß Figur 1 dadurch, dass anstatt einer einzigen Synthesestufe 22 stattdessen drei in Serie geschaltete Synthesestufen 22a, 22b und 22c vom Verfahren umfasst sind. Ferner wird der Frischgasstrom 11 vom Kompressor 12 nur auf 90 bar in Bezug auf den Druck des verdichteten Frischgasstrom MG 13 verdichtet. Die Rezirkulationsrate R beträgt aufgrund des Vorhandenseins dreier in Serie geschalteter Synthesestufen und des dadurch höheren Kohlenstoffumsatzes (Umsatz an im Synthesegas gebundenen Kohlenstoff zu in Methanol gebundenem Kohlenstoff) "pro Durchgang" nur 1,0.

Jede der Synthesestufen 22a, 22b und 22c verfügt über Mittel zum Kühlen, Kondensieren und Separieren von Rohmethanol. Dieses wird entsprechend als drei getrennte Rohmethanolströme 21a, 21b und 21c aus den jeweils zugehörigen Synthesestufen 22a, 22b und 22c ausgeleitet. Die drei Rohmethanolteilströme 21a, 21b und 21c werden anschließend zu einem Rohmethanolgesamtstrom 21 zusammengeführt. Der Rohmethanolstrom 21 wird einer weiteren Aufarbeitung zur Gewinnung von reinem Methanol zugeführt (nicht gezeigt). Von dem jeweiligen Rohmethanolstrom wird je Synthesestufe auch ein Strom von nicht umgesetztem Synthesegas abgetrennt. Im Falle der ersten Synthesestufe 22a wird das nicht umgesetzte Synthesegas als Zwischengasstrom IG 23a der zweiten Synthesestufe 22b zugeführt. Das in der zweiten Synthesestufe 22b nicht umgesetzte Synthesegas wird als Zwischengasstrom IG 23b der dritten und letzten Synthesestufe 22c zugeführt. Schließlich wird in der dritten Synthesestufe 22c nicht zu Methanol und Wasser umgesetztes Synthesegas als Restgasstrom RG 19 zum Saugstutzen 16 der Strahlpumpe 14 zurückgeführt und dort wiederum mit dem über den Treibmedienanschluss 15 zugeführten Frischgasstrom MG 13 zusammengeführt.

Je nach Verfahrensführung ist es auch möglich, dass aus den jeweiligen Zwischengasströmen IG 23a und/oder 23b Teilströme abgezweigt und nicht in die folgende Synthesestufe, sondern zum Treibmedienanschluss 15 der Strahlpumpe 14 zurückgeführt werden.

Um die Kumulation von unter den Bedingungen der Methanolsynthese inerten Edukt- oder Produktkomponenten in der Syntheseschleife zu verhindern, wird auch im Falle des Beispiels gemäß Figur 2 vom Restgasstrom RG 19 ein Purgegasstrom 20 abgeführt und einer weiteren Verwendung zugeführt.

Die Rezirkulationsrate R beträgt im Falle des Beispiels gemäß Figur 2 R = 1,0.

Die folgenden Zahlenbeispiele dienen der weiteren Erläuterung der Erfindung. Die Zahlenbeispiele wurden mit Hilfe der Simulationssoftware Aspen Plus® generiert. Dabei wird in den Beispielen von einem "worst case scenario" ausgegangen, nämlich den für die Methanolbildung sehr ungünstigen Fall eines Synthesegases, welches als Kohlenstoffoxid ausschließlich Kohlendioxid umfasst. In einem solchen Fall ist die erforderliche Rezirkulationsrate besonders hoch, da das thermodynamische Gleichgewicht stark auf der Eduktseite liegt und der Kohlenstoffumsatz (Umsatz von Kohlenstoff im Synthesegas zu Methanol) vergleichsweise niedrig ist.

Die folgende Tabelle zeigt einen Vergleich zwischen zwei Konfigurationen gemäß Stand der Technik und Erfindung für ein Verfahren mit einer einzigen Synthesestufe. Vergleichsbeispiel 1 entspricht einer Konfiguration mit dediziertem Restgaskompressor, Beispiel 1 entspricht einer Konfiguration mit Strahlpumpe wie in Figur 1 dargestellt.

| | Vergleichsbeispiel 1 (Restgaskompressor) | Beispiel 1 (Strahlpumpe) |
|---|---|---|
| Druck am Kompressoreinlass (Strom 11) / bar | 30 | 30 |
| Rezirkulationsrate | 4,6 | 4,6 |
| Druckabfall über Synthesestufe / bar | 2 | 2 |
| Druck am Kompressorauslass (Strom 13) / bar | 80 | 95 |
| Normalisierter Leistungsbedarf Verdichtungsarbeit Kompressor 12 | 100 % | 120 % |

Durch den Wegfall des Restgaskompressors gemäß Vergleichsbeispiel 1 und Ersatz dieses Restgaskompressors durch eine Strahlpumpe gemäß Beispiel 1 steigt der Leistungsbedarf des Kompressors 12 um 20 %. Insbesondere bei Anlagen im kleinen oder mittleren Maßstab können die damit verbundenen höheren Betriebskosten durch die gleichzeitig jedoch sinkenden Investitions- und Wartungskosten überkompensiert werden.

Die folgende Tabelle zeigt einen Vergleich zwischen zwei Konfigurationen gemäß Stand der Technik und Erfindung für ein Verfahren mit drei in Serie geschalteten Synthesestufen. Vergleichsbeispiel 2 entspricht einer Konfiguration mit dediziertem Restgaskompressor, Beispiel 2 entspricht einer Konfiguration mit Strahlpumpe wie in Figur 2 dargestellt.

| | Vergleichsbeispiel 2 (Restgaskompressor) | Beispiel 2 (Strahlpumpe) |
|---|---|---|
| Druck am Kompressoreinlass (Strom 11) / bar | 30 | 30 |
| Rezirkulationsrate | 1,0 | 1,0 |
| Druckabfall über Synthesestufe / bar | 6 | 6 |
| Druck am Kompressorauslass (Strom 13) / bar | 80 | 90 |
| Normalisierter Leistungsbedarf Verdichtungsarbeit Kompressor 12 | 100 % | 112 % |

Da es sich gemäß Vergleichsbeispiel 2 und Beispiel 2 um eine dreistufige Synthese mit Zwischenkondensation des Rohmethanols handelt, ist der Kohlenstoffumsatz "pro Durchgang" höher, und die Rezirkulationsrate entsprechend um ein mehrfaches niedriger als gemäß Vergleichsbeispiel 1 und Beispiel 1. Der Druckabfall über die Synthesestufen beträgt aufgrund der dreifachen Anzahl an Synthesestufen 6 bar statt 2 bar.

Durch den Wegfall des Restgaskompressors gemäß Vergleichsbeispiel 2 und Ersatz dieses Restgaskompressors durch eine Strahlpumpe gemäß Beispiel 2 steigt der Leistungsbedarf des Kompressors 12 um lediglich 12 %. Dies ist auch in Anbetracht der Tatsache eine überraschende Feststellung, da der Druckabfall im Fall der Mehrstufensynthese gemäß Figur 2 dreimal so hoch ist im Vergleich zur Einstufensynthese gemäß Figur 1, was durch Verdichtung kompensiert werden muss. In diesem Fall hat die niedrige Rezirkulationsrate von 1,0 somit bezüglich des erforderlichen Kompressor-Leistungsbedarfs trotz höheren Druckabfalls in der Syntheseschleife einen überkompensierenden Effekt.

Synthesegase mit hohem Kohlenmonoxidanteil haben auch in einstufigen Konfigurationen einen hohen Kohlenstoffumsatz "pro Durchgang" zur Folge, wodurch entsprechend niedrigere Rezirkulationsraten erforderlich sind. Der vorgenannten Effekt sollte daher insbesondere auch bei einstufigen oder zweistufigen Verfahren mit niedrigen Rezirkulationsraten, welche einen niedrigeren Druckabfall über die Synthesestufen aufweisen, zum Tragen kommen.

**Bezugszeichenliste**

| | |
|---|---|
| 1, 2 | Verfahren, Anlage |
| 10 | Vorrichtung zur Synthesegaserzeugung |
| 11 | Frischgasstrom |
| 12 | Kompressor |
| 13 | verdichteter Frischgasstrom MG |

| | |
|---|---|
| 14 | Strahlpumpe |
| 15 | Treibmedienanschluss |
| 16 | Saugstutzen |
| 17 | Druckstutzen |
| 18 | Synthesegasstrom SG |
| 19 | Restgasstrom RG |
| 20 | Purgegasstrom |
| 21, 21a, 21b, 21c | Rohmethanolstrom |
| 22, 22a, 22b, 22c | Synthesestufe oder Reaktorstufe |
| 23a, 23b | Zwischengasstrom IG |
| $p_1$, $p_2$, $p_3$ | Mess- und Regelstellen für Druck |

## Patentansprüche

**1.** Verfahren (1, 2) zur Herstellung von Methanol, wobei das Verfahren die folgenden Schritte umfasst:

a) Bereitstellen und Verdichten eines Frischgasstroms MG (13), wobei der Frischgasstrom MG (13) zumindest ein Kohlenstoffoxid und Wasserstoff aufweist;
b) Umsetzen eines Synthesegasstroms SG (18) in einer Synthesestufe (22, 22a, 22b, 22c) an einem festen Methanolsynthese-Katalysator, wobei Rohmethanol und ein Restgasstrom RG (19) erhalten werden, und wobei das Rohmethanol nach Kühlung und Kondensation als flüssiges Reaktionsprodukt (21, 21a, 21b, 21c) und der Restgasstrom RG (19) aus der Synthesestufe (22, 22a, 22b, 22c) ausgeleitet werden;
c) Zusammenführen des Restgasstroms RG (19) mit dem Frischgasstrom MG (13), wobei der Synthesegasstrom SG (18) erhalten wird,

**dadurch gekennzeichnet, dass**
der Restgasstrom RG (19) und der Frischgasstrom MG (13) mittels einer Strahlpumpe (14) zusammengeführt werden, wobei der verdichtete Frischgasstrom MG (13) mit einem Druck $p_1$ der Strahlpumpe über deren Treibmedienanschluss (15) als Treibmedium zugeführt wird, und der Restgasstrom RG (19) mit einem Druck $p_3$ der Strahlpumpe über deren Saugstutzen (16) als Saugmedium zugeführt wird, und wobei der Synthesegasstrom SG (18) mit einem Druck $p_2$ aus der Strahlpumpe über deren Druckstutzen (18) ausgeleitet und anschließend der Synthesestufe (22, 22a) zugeführt wird, und wobei $p_1 > p_2 > p_3$ gilt.

**2.** Verfahren nach Anspruch 1, **dadurch gekennzeichnet, dass** für eine Rezirkulationsrate R, definiert als

$$R = \frac{Volumenstrom\ (RG)}{Volumenstrom\ (MG)},$$

$0{,}3 \leq R \leq 2{,}0$ gilt, vorzugsweise $0{,}5 \leq R \leq 1{,}5$ gilt, weiter bevorzugt $1{,}0 \leq R \leq 1{,}5$ gilt.

**3.** Verfahren nach Anspruch 1 oder 2, **dadurch gekennzeichnet, dass** das Verfahren eine Vielzahl N in Serie geschalteter Synthesestufen (22a, 22b, 22c) beinhaltet, wobei das Rohmethanol nach Kühlung und Kondensation als flüssiges Reaktionsprodukt (21a, 21b, 21c) aus jeder der Synthesestufen ausgeleitet wird, und der Restgasstrom RG (19) aus der letzten der N Synthesestufen (22c) ausgeleitet wird.

**4.** Verfahren nach Anspruch 3, **dadurch gekennzeichnet, dass** $N \geq 2$ gilt, vorzugsweise $2 \leq N \leq 5$ gilt, weiter bevorzugt $3 \leq N \leq 4$ gilt.

**5.** Verfahren nach einem der vorhergehenden Ansprüche, **dadurch gekennzeichnet, dass** der Anteil an Kohlendioxid an den Kohlenoxiden im Frischgasstrom MG mindestens 50 Vol.-% beträgt, oder mindestens 75 Vol.-% beträgt, oder mindestens 90 Vol.-% beträgt.

**6.** Anlage (1, 2) zur Herstellung von Methanol, wobei die Anlage folgende untereinander in Wirkverbindung stehende Anlagenkomponenten aufweist:

a) Einen Kompressor (12) zum Verdichten eines Frischgasstroms MG (13), wobei der Frischgasstrom MG (13)

zumindest ein Kohlenstoffoxid und Wasserstoff aufweist;
b) Eine Reaktionsvorrichtung zum Umsetzen eines Synthesegasstroms SG (18) in einer Reaktorstufe (22, 22a, 22b, 22c) an einem festen Methanolsynthese-Katalysator, wobei Rohmethanol und ein Restgasstrom RG (19) durch die Umsetzung an dem Methanolsynthese-Katalysator erhältlich sind, und wobei die Reaktorstufe (22, 22a, 22b, 22c) Mittel zum Kühlen und Kondensieren des Rohmethanols aufweist, sowie Mittel zum Ausleiten des Rohmethanols als flüssiges Reaktionsprodukt (21, 21a, 21b, 21c) und des Restgasstroms (19) aus der Reaktorstufe aufweist;
c) Mittel zum Zusammenführen des Restgasstroms RG (19) und des Frischgasstroms MG (13), wodurch der Synthesegasstrom SG (18) erhältlich ist,

**dadurch gekennzeichnet, dass**

die Anlage eine Strahlpumpe (14) als Mittel zum Zusammenführen des Restgasstroms RG (19) und des Frischgasstrom MG (13) aufweist, und
die Anlage Mittel zum Zuführen des verdichteten Frischgasstroms MG (13) mit einem Druck $p_1$ zu einem Treibmedienanschluss (15) der Strahlpumpe (14) aufweist, wodurch der Frischgasstrom MG (13) als Treibmedium der Strahlpumpe nutzbar ist, und die Anlage Mittel zum Zuführen des Restgasstroms RG (19) mit einem Druck $p_3$ zu einem Saugmedienanschluss (16) der Strahlpumpe (14) aufweist, und der Synthesegasstrom SG (18) mit einem Druck $p_2$ über einen Druckstutzen (17) der Strahlpumpe (14) aus der Strahlpumpe ausleitbar und der Reaktionsvorrichtung zuführbar ist, und wobei $p_1 > p_2 > p_3$ gilt.

7. Anlage nach Anspruch 6, **dadurch gekennzeichnet, dass** die Reaktionsvorrichtung der Anlage eine Vielzahl P von in Serie geschalteten Reaktorstufen (22a, 22b, 22c) aufweist, wobei das Rohmethanol nach Kühlung und Kondensation als flüssiges Reaktionsprodukt (21a, 21b, 21c) aus jeder der Reaktorstufen (22a, 22b, 22c) ausleitbar ist, und der Restgasstrom RG (19) aus der letzten der P Reaktorstufen (22c) ausleitbar ist.

8. Anlage nach Anspruch 7, **dadurch gekennzeichnet, dass** $P \geq 2$ gilt, vorzugsweise $2 \leq P \leq 5$ gilt, weiter bevorzugt $3 \leq P \leq 4$ gilt.

9. Anlagen nach einem der Ansprüche 6 bis 8, **dadurch gekennzeichnet, dass** die Anlage so konfiguriert ist, dass sie mit einer Rezirkulationsrate R, definiert als

$$R = \frac{Volumenstrom\ (RG)}{Volumenstrom\ (MG)},$$

betreibbar ist und $0{,}3 \leq R \leq 2{,}0$ gilt, vorzugsweise $0{,}5 \leq R \leq 1{,}5$ gilt, weiter bevorzugt $1{,}0 \leq R \leq 1{,}5$ gilt.

## Claims

1. Process (1, 2) for producing methanol, wherein the process comprises the steps of:

a) providing and compressing a make-up gas stream MG (13), wherein the make-up gas stream MG (13) comprises at least one carbon oxide and hydrogen;
b) reacting a synthesis gas stream SG (18) in a synthesis stage (22, 22a, 22b, 22c) over a solid methanol synthesis catalyst, wherein raw methanol and a residual gas stream RG (19) are obtained and wherein the raw methanol after cooling and condensation as liquid reaction product (21, 21a, 21b, 21c) and the residual gas stream RG (19) are discharged from the synthesis stage (22, 22a, 22b, 22c);
c) combining the residual gas stream RG (19) with the make-up gas stream MG (13) to obtain the synthesis gas stream SG (18),

**characterized in that**
the residual gas stream RG (19) and the make-up gas stream MG (13) are combined using a jet pump (14), wherein the compressed make-up gas stream MG (13) is supplied to the jet pump as motive medium via its motive media connection (15) at a pressure $p_1$ and the residual gas stream RG (19) is supplied to the jet pump as suction medium via its suction port (16) at a pressure $p_3$ and wherein the synthesis gas stream SG (18) is discharged from the jet pump via its pressure port (18) at a pressure $p_2$ and subsequently supplied to the synthesis stage (22, 22a) and

wherein $p_1 > p_2 > p_3$.

2. Process according to Claim 1, **characterized in that** for a recirculation rate R defined as

$$R = \frac{Volume\ flow\ (RG)}{Volume\ flow\ (MG)},$$

$0.3 \leq R \leq 2.0$, preferably $0.5 \leq R \leq 1.5$, more preferably $1.0 \leq R \leq 1.5$.

3. Process according to Claim 1 or 2, **characterized in that** the process comprises a plurality N of serially arranged synthesis stages (22a, 22b, 22c), wherein the raw methanol after cooling and condensation as liquid reaction product (21a, 21b, 21c) is discharged from each of the synthesis stages and the residual gas stream RG (19) is discharged from the last of the N synthesis stages (22c) .

4. Process according to Claim 3, **characterized in that** $N \geq 2$, preferably $2 \leq N \leq 5$, more preferably $3 \leq N \leq 4$.

5. Process according to any of the preceding claims, **characterized in that** the proportion of carbon dioxide in the carbon oxides in the make-up gas stream MG is at least 50% by volume or at least 75% by volume or at least 90% by volume.

6. Plant (1, 2) for producing methanol, wherein the plant comprises the following components in fluid connection with one another:

a) a compressor (12) for compressing a make-up gas stream MG (13), wherein the make-up gas stream MG (13) comprises at least one carbon oxide and hydrogen;
b) a reaction apparatus for reacting a synthesis gas stream SG (18) in a reactor stage (22, 22a, 22b, 22c) over a solid methanol synthesis catalyst, wherein raw methanol and a residual gas stream RG (19) are obtainable by the reaction over the methanol synthesis catalyst and wherein the reactor stage (22, 22a, 22b, 22c) comprises means for cooling and condensing the raw methanol and means for discharging the raw methanol as liquid reaction product (21, 21a, 21b, 21c) and the residual gas stream (19) from the reactor stage;
c) means for combining the residual gas stream RG (19) and the make-up gas stream MG (13) by which the synthesis gas stream SG (18) is obtainable,

**characterized in that**
the plant comprises a jet pump (14) as means for combining the residual gas stream RG (19) and the make-up gas stream MG (13) and the plant comprises means for supplying the compressed make-up gas stream MG (13) to a motive media connection (15) of the jet pump (14) at a pressure $p_1$, thus making the make-up gas stream MG (13) employable as the motive medium of the jet pump, and the plant comprises means for supplying the residual gas stream RG (19) to a suction media connection (16) of the jet pump (14) at a pressure $p_3$ and the synthesis gas stream SG (18) is dischargeable from the jet pump and suppliable to the reaction apparatus via a pressure port (17) of the jet pump (14) at a pressure $p_2$ and wherein $p_1 > p_2 > p_3$.

7. Plant according to Claim 6, **characterized in that** the reaction apparatus of the plant comprises a plurality P of serially arranged reactor stages (22a, 22b, 22c), wherein the raw methanol after cooling and condensation as liquid reaction product (21a, 21b, 21c) is dischargable from each of the reactor stages (22a, 22b, 22c) and the residual gas stream RG (19) is dischargable from the last of the P reactor stages (22c).

8. Plant according to Claim 7, **characterized in that** $P \geq 2$, preferably $2 \leq P \leq 5$, more preferably $3 \leq P \leq 4$.

9. Plant according to any of Claims 6 to 8, **characterized in that** the plant is configured such that it is operable at a recirculation rate R defined as

$$R = \frac{Volume\ flow\ (RG)}{Volume\ flow\ (MG)},$$

and $0.3 \leq R \leq 2.0$, preferably $0.5 \leq R \leq 1.5$, more preferably $1.0 \leq R \leq 1.5$.

## Revendications

1. Procédé (1, 2) pour la production de méthanol, le procédé comprenant les étapes suivantes :

   a) fourniture et compression d'un courant de gaz neuf MG (13), le courant de gaz neuf (13) comportant au moins un oxyde de carbone et de l'hydrogène ;
   b) mise en réaction d'un courant de gaz de synthèse SG (18) dans un étage de synthèse (22, 22a, 22b, 22c) sur un catalyseur solide pour synthèse de méthanol, avec obtention de méthanol brut et d'un courant de gaz résiduaire RG (19), et le méthanol après refroidissement et condensation, en tant que produit liquide de réaction (21, 21a, 21b, 21c), et le courant de gaz résiduaire RG (19) étant évacués de l'étage de synthèse (22, 22a, 22b, 22c) ;
   c) réunion du courant de gaz résiduaire RG (19) avec le courant de gaz neuf MG (13), avec obtention du courant de gaz de synthèse SG (18),

   **caractérisé en ce que**
   le courant de gaz résiduaire RG (19) et le courant de gaz neuf MG (13) sont réunis au moyen d'une pompe à jet (14), le courant de gaz neuf (13) comprimé étant envoyé en tant que milieu propulseur sous une pression $p_1$ à la pompe à jet via son raccord (15) de milieu propulseur, et le courant de gaz résiduaire RG (19) étant envoyé en tant que milieu aspiré sous une pression $p_3$ à la pompe à jet via sa tubulure d'aspiration (16), et le courant de gaz de synthèse SG (18) étant évacué sous une pression $p_2$ de la pompe à jet via sa tubulure de refoulement et ensuite envoyé à l'étage de synthèse (22, 22a), et où il s'applique que $p_1 > p_2 > p_3$.

2. Procédé selon la revendication 1, **caractérisé en ce que** pour un taux de recirculation R, défini comme

   $$R = \frac{débit\ volumique\ (RG)}{débit\ volumique\ (MG)},$$

   il s'applique que $0{,}3 \leq R \leq 2{,}0$, de préférence $0{,}5 \leq R \leq 1{,}5$, encore mieux $1{,}0 \leq R \leq 1{,}5$.

3. Procédé selon la revendication 1 ou 2, **caractérisé en ce que** le procédé comporte une pluralité N d'étages de synthèse (22a, 22b, 22c) raccordés en série, le méthanol brut, après refroidissement et condensation, étant évacué, en tant que produit liquide de réaction (21a, 21b, 21c), de chacun des étages de synthèse, et le courant de gaz résiduaire RG (19) étant évacué du dernier des N étages de synthèse (22c).

4. Procédé selon la revendication 3, **caractérisé en ce qu'**il s'applique que $N \geq 2$, de préférence $2 \leq N \leq 5$, encore mieux $3 \leq N \leq 4$.

5. Procédé selon l'une quelconque des revendications précédentes, **caractérisé en ce que** la teneur en dioxyde de carbone des oxydes de carbone dans le courant de gaz neuf MG vaut au moins 50 % en volume, ou vaut au moins 75 % en volume, ou vaut au moins 90 % en volume.

6. Installation (1, 2) destinée à la production de méthanol, l'installation comportant les composants d'installation suivants en connexion fonctionnelle :

   a) un compresseur (12) destiné à la compression d'un courant de gaz neuf MG (13), le courant de gaz neuf MG (13) comportant au moins un oxyde de carbone et de l'hydrogène ;
   b) un dispositif de réaction destiné à la mise en réaction d'un courant de gaz de synthèse SG (18) dans un étage réacteur (22, 22a, 22b, 22c) sur un catalyseur solide pour synthèse de méthanol, du méthanol brut et un courant de gaz résiduaire RG (19) pouvant être obtenus par la réaction sur le catalyseur pour synthèse de méthanol, et l'étage réacteur (22, 22a, 22b, 22c) comportant des moyens destinés au refroidissement et à la condensation du méthanol brut, ainsi que des moyens destinés à l'évacuation du méthanol brut en tant que produit liquide de réaction (21, 21a, 21b, 21c), et du courant de gaz résiduaire RG (19) hors de l'étage réacteur ;
   c) des moyens destinés à la réunion du courant de gaz résiduaire RG (19) avec le courant de gaz neuf MG (13),ce par quoi le courant de gaz de synthèse SG (18) peut être obtenu,

   **caractérisée en ce que**
   l'installation comporte une pompe à jet (14) en tant que moyen destiné à la réunion du courant de gaz résiduaire

RG (19) et du courant de gaz neuf MG (13), et l'installation comporte des moyens destinés à l'envoi du courant de gaz neuf MG (13) comprimé, sous une pression $p_1$, à un raccord (15) de milieu propulseur de la pompe à jet (14), ce par quoi le courant de gaz neuf MG (13) est utilisable en tant que milieu propulseur de la pompe à jet, et l'installation comporte des moyens destinés à l'envoi du courant de gaz résiduaire RG (19) sous une pression $p_3$ à un raccord (16) de milieu aspiré de la pompe à jet (14), et le courant de gaz de synthèse SG (18) peut être évacué de la pompe à jet sous une pression $p_2$ via une tubulure de refoulement (17) de la pompe à jet (14) et peut être envoyé au dispositif de réaction, et où il s'applique que $p_1 > p_2 > p_3$.

**7.** Installation selon la revendication 6, **caractérisée en ce que** le dispositif de réaction de l'installation comporte une pluralité P d'étages réacteurs (22a, 22b, 22c) raccordés en série, le méthanol brut, après refroidissement et condensation, étant évacué en tant que produit liquide de réaction (21a, 21b, 21c), de chacun des étages réacteurs (22a, 22b, 22c), et le courant de gaz résiduaire RG (19) étant évacué du dernier des P étages réacteurs (22c).

**8.** Installation selon la revendication 7, **caractérisée en ce qu'**il s'applique que $P \geq 2$, de préférence $2 \leq P \leq 5$, encore mieux $3 \leq P \leq 4$.

**9.** Installation selon l'une quelconque des revendications 6 à 8, **caractérisée en ce que** l'installation est configurée de telle façon qu'elle peut être mise en fonction à un taux de recirculation R, défini comme

$$R = \frac{débit\ volumique\ (RG)}{débit\ volumique\ (MG)},$$

et il s'applique que $0{,}3 \leq R \leq 2{,}0$, de préférence $0{,}5 \leq R \leq 1{,}5$, encore mieux $1{,}0 \leq R \leq 1{,}5$.

Fig. 1

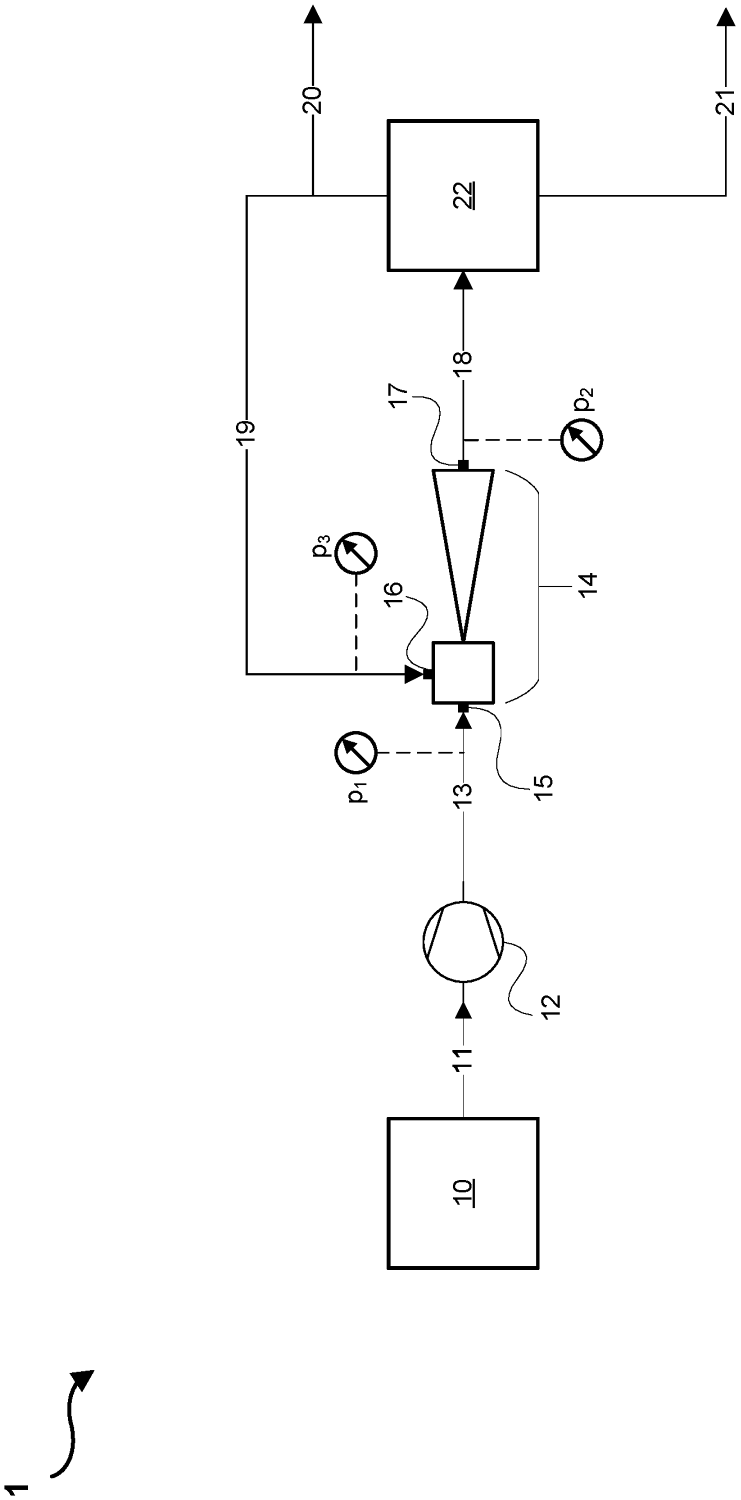

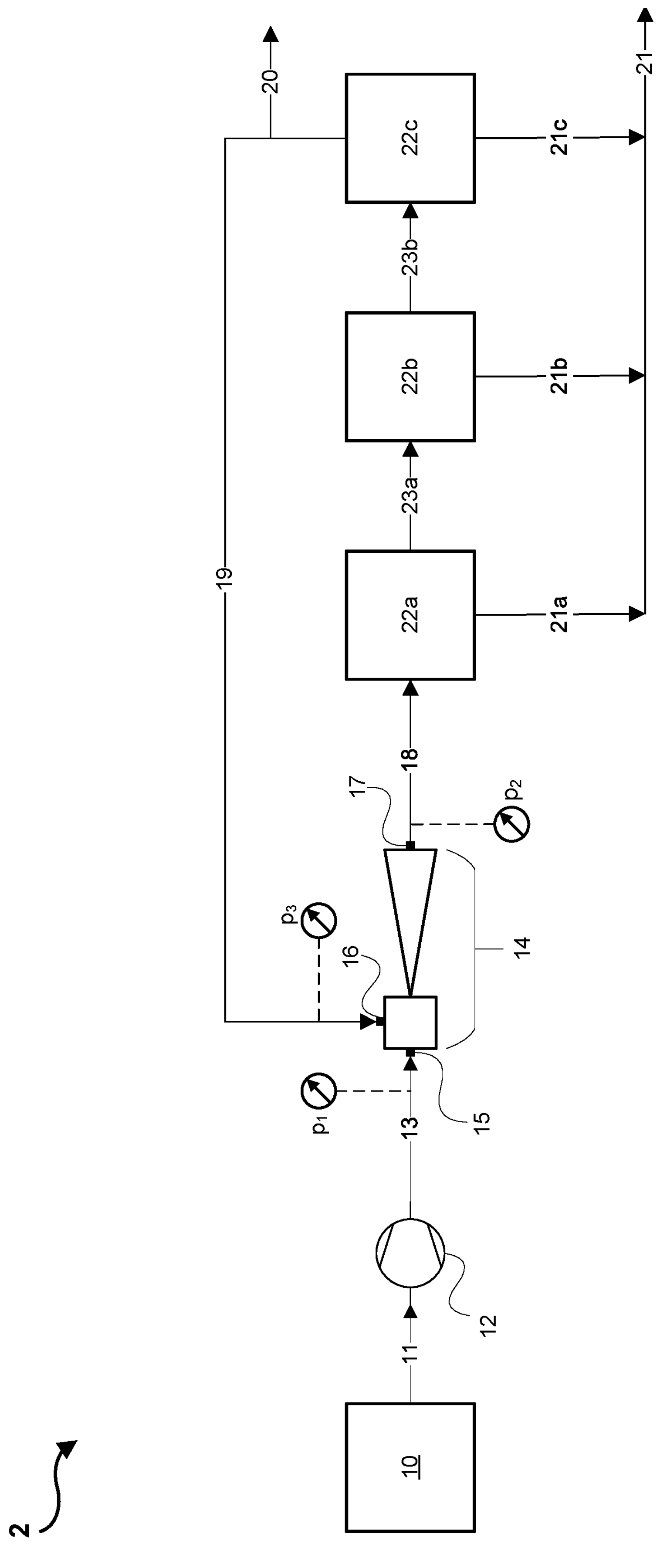

2
10
11
12
13
$p_1$
15
14
16
$p_3$
17
$p_2$
18
19
22a
21a
23a
22b
21b
23b
22c
21c
20
21


Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 3401299 A1
- EP 3401300 A1